# EUROPÄISCHE PATENTSCHRIFT

(11) **EP 1 013 236 B1**
(45) Veröffentlichungstag und Bekanntmachung des Hinweises auf die Patenterteilung: **08.11.2000**
(21) Anmeldenummer: 98123681.3
(22) Anmeldetag: 11.12.1998
(51) Int. Cl.: A61C 8/00

(54) **Zahnimplantat und Verfahren zu seiner Herstellung**
Dental implant and manufacturing method
Implant dentaire et son procédé de fabrication

(43) Veröffentlichungstag der Anmeldung: 28.06.2000
(73) Patentinhaber: Dinkelacker, Wolfgang, Dr. med. dent., 71063 Sindelfingen (DE)
(72) Erfinder: Dinkelacker, Wolfgang, Dr. med. dent., 71063 Sindelfingen (DE)
(74) Vertreter: Kindermann, Manfred

(56) Entgegenhaltungen:
- DE-A- 3 811 498
- DE-A- 4 130 891
- DE-U- 29 707 545
- FR-A- 2 715 568
- US-A- 5 820 374

## Beschreibung

### Bereich der Erfindung

Die Erfindung bezieht sich auf ein operativ in den Kiefer einsetzbares Zahnimplantat mit einem rotationssymmetrischen Implantatkörper, der eine gerauhte Oberfläche aufweist, sowie auf ein Verfahren zur Herstellung des Implantats.

### Stand der Technik

Bekannte Zahnimplantate weisen einen rotationssymmetrischen Implantatkörper auf, der als Zylinder oder Konus ausgebildet sein kann oder eine Stufenform besitzt. Der Implantatkörper besteht aus Titan, das eine gute biologische Verträglichkeit mit dem Knochengewebe zeigt. Die Oberfläche des Implantatkörpers weist in dem mit Körpergewebe in Kontakt kommenden Bereich eine die stabile Einheilung fördernde Oberfläche auf, wie z.B.eine Beschichtung mit Hydroxylapatit (DE-A 38 39 724). Es ist auch bekannt, die Oberfläche des Implantatkörpers nach einer Entfernung der natürlichen Titanoxydschicht einem Säureätzprozess auszusetzen, um eine im wesentlichen gleichförmige Rauhigkeit über die ganze Oberfläche zu erzielen (WO 96/16611).

Ein Zahnimplantat gemäß dem Oberbegriff des Anspruchs 1 ist in US-A-5820374 offenbart. Dieses Implantat besitzt einen zylindrischen Körper, der an seiner Oberfläche diamantförmigen Erhebungen aufweist. Diese Erhebungen werden durch im spitzen Winkel zur Längsachse des Implantats verlaufende Nuten oder Rillen gebildet, die durch diagonale Rändelung, Riffelung oder Kordelung hergestellt werden. Diese Oberflächenstruktur erstreckt sich einheitlich und ohne Unterbrechungen über die ganze Länge des zylindrischen Implantatkörpers.

Durch die Behandlung der Oberfläche des Implantatkörpers soll der Kontakt mit dem umgebenden Knochengewebe erleichtert und die daran beteiligte Implantatoberfläche vergrößert werden. Hierzu ist eine Titan-Plasmabeschichtung (TPS) bekannt, die durch Thermospritzen von Titan auf das Titanimplantat erzeugt wird. Des weiteren wird ein Verfahren angewendet, das zunächst eine Behandlung der Oberfläche durch Grobsandstrahlen vorsieht, wodurch eine Makrorauheit auf dem Titan erzielt wird. Diesem Vorgang folgt eine Säureätzung, durch die über die sandgestrahlten Oberfläche gleichförmig verteilte Mikrogrübchen erzeugt werden (Cochran et al, "Bone response to unloaded and loaded titanium implants with a sand blasted and acid-etched surface", Jounal of Biomedical Materials Research, Vol. 40, 1998, S. 1).

Bei den bekannten Oberflächenausbildungen ist eine Anlagerung der Osteone des Knochens an der Oberfläche des Implantatkörpers nicht oder nur in einem beschränkten Umfang möglich. Hierdurch wird eine biomechanische Integration des Implantats behindert.

Ziel der Erfindung ist es, die Oberfläche des Implantats derart zu gestalten, daß eine verbesserte Knochenanlagerung und damit eine dauernde biomechanische Integration des Implantats im Kieferknochen erfolgt. Gegenstand der Erfindung ist des weiteren ein Verfahren zur Herstellung einer solchen Oberfläche.

### Zusammenfassung der Erfindung

Nach der Erfindung, wie sie in den Ansprüchen 1 und 21 definiert ist, wird die Oberfläche des Implantatkörpers mit einer Vielzahl in Richtung seiner Längsachse oder in einem spitzen Winkel zu dieser verlaufende rillenförmige Vertiefungen ausgestattet.

Die Histologie des Knochens weist eine Lamellenstruktur auf, die sich zusammensetzt aus einer äußeren Generallamelle mit darin eingebetteten Osteozyten, welche eine Größe von 2 bis 3 Mikrometern aufweisen, aus Osteonen (Havers-Systeme) mit Speziallamellen von einer Größe zwischen 20 bis 300 Mikrometern, aus Schaltlamellen, die abgestorbene und zum Teil umgewandelte Osteone enthalten, und aus einer inneren Generallamelle. Die Osteone sind mit Kollagen umgeben, die sich schraubenförmig um den Havers-Kanal bilden wie eine Baumstruktur und in die Knochensubstanz eingebettet sind. Die Osteone sind nach den statischen Aufgaben des Knochens ausgerichtet.

Durch die Erfindung wird es dem Knochen ermöglicht, daß sich seine Osteone in den rillenförmigen Vertiefungen der Implantatoberfläche anlagern und entlang des Implantats wachsen. Die von den rillenförmigen Vertiefungen gebildeten Furchen werden in ihrer Dimension an die Größenordnung der Osteone angepaßt und nehmen die mit dem Implantat in Kontakt kommenden Teile der Osteone auf. Als Folge hiervon und durch die auf das Implantat einwirkenden Drücke bildet sich im Spongiosa-Bereich des Knochens eine Compakta um das Implantat aus, die eine verbesserte Aufnahme der auf das Implantat einwirkenden Kräfte und einen stabilen und dauerhaften Sitz des Implantats bewirkt.

Diese Wirkung wird dadurch noch verstärkt, daß in den rillenförmigen Vertiefungen eine Vielzahl von in Bezug auf diese klein ausgebildeten kallotenartigen Vertiefungen dicht nebeneinander und räumlich verteilt angeordnet sind. Die kallotenartigen Vertiefungen nehmen die Osteozyten des das Implantat umgebenden Kochengewebes auf und unterstützen damit den Kontakt zwischen Implantat und Knochen.

Eine Verbesserung der Osteonen-Anlagerung wird weiter durch Anordnung von kreuzweise verlaufenden rillenförmigen Vertiefungen erreicht. Hierzu sind erste Rillen vorgesehen, die in einer ersten Richtung in einem spitzen Winkel zur Längsachse des Implantatkörpers verlaufen, und zweite Rillen vorgesehen, die in einer zweiten Richtung in einem spitzen Winkel zur Längsachse des Implantatkörpers verlaufen und die sich mit den ersten Rillen kreuzen. Die rillenförmigen Vertiefungen sind vorzugsweise einander dicht benachbart und weisen ein konkaves Profil auf, das an seinen Rändern in den Umfang des Implantatkörpers ausläuft. Eine solche Oberflächenstruktur bietet günstige Vorraussetzungen für die Anlagerung der Osteone während der Einheilphase und bewirkt eine zusätzliche Sicherung gegen axiale Verlagerung und Verdrehung des Implantats im eingeheilten Zustand.

Des weiteren ist die Oberfläche des Implantatkörpers in Richtung seiner Längsachse in eine Anzahl Abschnitte unterteilt, die durch radial verlaufende Bänder voneinander getrennt sind. Die Abschnitte weisen an ihrem Umfang eine Vielzahl in Richtung der Längsachse des Implantatkörpers oder in einem spitzen Winkel zu dieser verlaufende rillenförmige Vertiefungen auf. Die Bänder übernehmen während der Einheilphase und im eingeheilten Zustand die Funktion von Barrieren gegen eventuelle vom Kopf des Implantats nach unten absteigende Infektionsherde. Der Durchmesser der Abschnitte kann unterschiedlich sein und zum unteren Endes des Implantatkörpers stufenförmig abnehmen, wobei sich der Durchmesser des Implantatkörpers jeweils im Bereich der radial verlaufenden Bänder verjüngt. Ebenso kann die Oberflächenstruktur der Abschnitte unterschiedlich sein. Diese Merkmale tragen wesentlich zu einem festen Sitz des Implantats im Kieferknochen bei und bieten gute Voraussetzungen für die Anlagerung der Osteonen in den Rillen eines jeden der Abschnitte.

Ein Verfahren zur Herstellung eines Zahnimplantats nach der Erfindung sieht vor, daß in einen in der gewünschten Form vorgeformten glatten Implantatkörper durch eine Materialabtragungsoperation eine Vielzahl in Richtung der Längsachse verlaufender rillenförmige Vertiefungen in die Oberfläche des Implantatkörpers eingebracht werden. Die Materialabtragungsoperation wird vorzugsweise durch einen digital gesteuerten Laserstrahl ausgeführt. Anstelle des Laserstrahls kann ein fokussierter Ionenstrahl Anwendung finden. Alternativ kann die Materialabtragungsoperation auch durch eine feinmechanische Fräßmaschine ausgeführt werden.

In einem weiteren Schritt werden Mulden oder Lagunen, die gegenüber den rillenförmigen Vertiefungen klein sind, in die Oberfläche des Implantatkörpers eingebracht. Dieser Schritt kann aus einer Säureätzung bestehen, wobei der Kopfbereich und in bestimmten Fällen auch der Fußbereich des Implantats jeweils mit einer säureresistenten Schicht abgedeckt wird. Zur Einbringung der Mulden oder Lagunen kann aber auch ein digital gesteuerter Laserstrahl verwendet werden, so daß für die Herstellung der rillenförmigen Vertiefungen und der Mulden oder Lagunen das gleiche Materialabtragungsverfahren Anwendung finden kann.

### Beschreibung der Zeichnungen

Nachfolgend sind verschiedene Ausführungsformen der Erfindung anhand von Zeichnungen dargestellt. Es zeigen:
- Figur 1: Beispiel eines zylindrischen Zahnimplantates mit axial verlaufenden Rillen,
- Figur 2: einen Teilschnitt entlang der Linie 2-2' in Figur 1,
- Figur 3: eine vergrößerte Ansicht eines Teils von Figur 1,
- Figur 4: einen Teilschnitt entlang der Linie 4-4' in Figur 3,
- Figur 5: eine vergrößerte Ansicht eines weiteren Teils von Figur 1,
- Figur 6: einen Teilschnitt entlang der Linie 6-6' in Figur 3,
- Figuren 7A, 7B und 7C: vergrößertes Draufsichten auf die rillenförmigen Vertiefungen, wie sie im Zahnimplantat nach den Figur 1 zur Anwendung kommen,
- Figur 8: Beispiel eines kegelförmigen Zahnimplantates mit kreuzweise verlaufenden Rillen,
- Figur 9: ein erfindungsgemäßes Zahnimplantat in einer abgestuften Kegelform mit axial verlaufenden Rillen,
- Figur 10: ein erfindungsgemäßes Zahnimplantat in einer abgestuften Kegelform mit kreuzweise verlaufenden Rillen,
- Figur 11: ein erfindungsgemäßes zylindrisches Zahnimplantat mit in Abschnitte unterteilten axial verlaufenden Rillen,
- Figuren 12 und 13: kegelförmige erfindungsgemäße Zahnimplantate mit in Abschnitte unterteilten axial verlaufenden Rillen bzw. kreuzweise verlaufenden Rillen,
- Figur 14: ein erfindungsgemäßes Zahnimplantat in einer abgestuften Kegelform mit axial verlaufenden Rillen im Bereich der einzelnen Stufen,
- Figur 15: ein erfindungsgemäßes Zahnimplantat in einer abgestuften Kegelform mit in einem spitzen Winkel zur Längsachse des Implantats verlaufenden Rillen am Umfang der einzelnen Stufen,
- Figur 16: erfindungsgemäßes ein Zahnimplantat in einer abgestuften Kegelform mit kreuzweise verlaufenden Rillen am Umfang der einzelnen Stufen,
- Figur 17: ein erfindungsgemäßes Zahnimplantat in einer abgestuften Kegelform mit unterschiedlich verlaufenden Oberflächenstrukturen im Bereich der einzelnen Stufen,
- Figur 18: ein erfindungsgemäßes Zahnimplantat in einer abgestuften Kegelform nach dem Einheilen in den Kieferknochen.

### Detaillierte Beschreibung der in den Zeichnungen dargestellten Ausführungsbeispiele der Erfindung

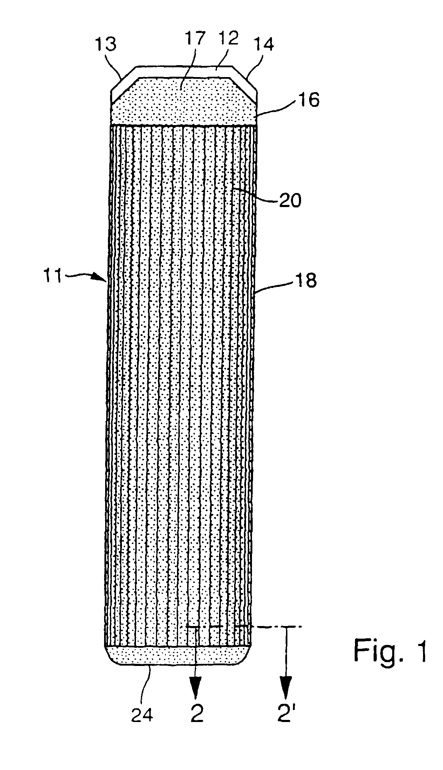

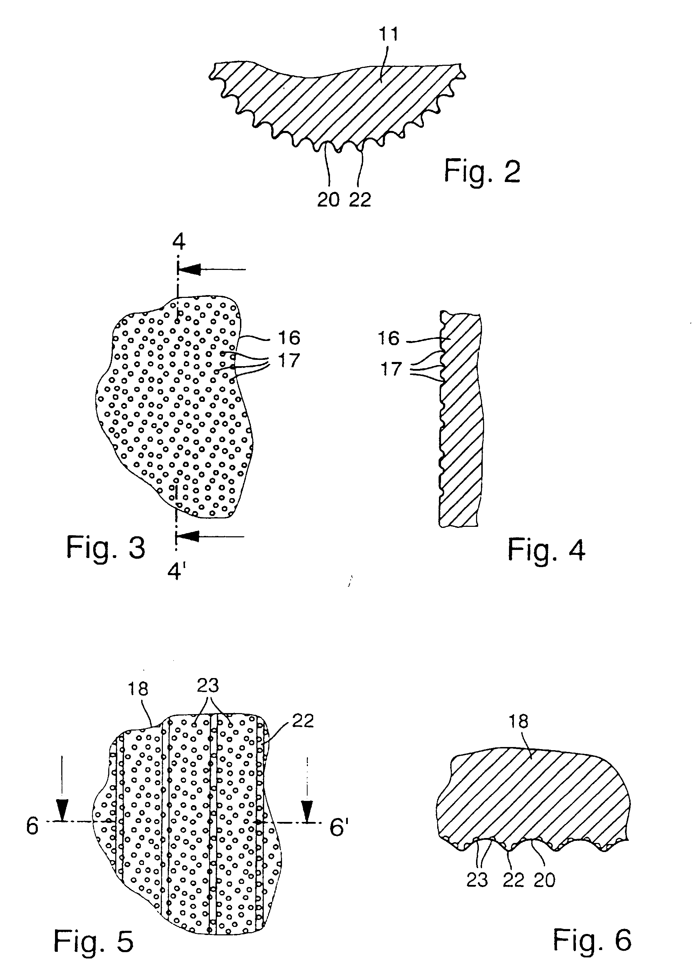

Das in Figur 1 dargestellte Zahnimplantat dient der Erläuterung der Oberflächenstruktur, es zeigt jedoch nicht das erfindungswesentliche Merkmal einer Unterteilung in durch radiale Bänder voneinander getrennte Längsabschnitte. Es umfaßt einen aus Titan bestehenden Zylinder 11, der einen Kopfbereich 12 mit einer polierten Oberfläche aufweist. Der Kopfbereich 12 kann mit Abschrägungen 13, 14 auf der Bucal- und Lingualseite ausgestattet sein, wie in der EP-A 0 868 889 dargestellt, und zur Aufnahme nichtgezeigter Tragelemente für eine Zahnkrone dienen. Unterhalb des Kopfbereichs 12 befindet sich ein Kragenbereich 16, der an seinem Umfang eine Vielzahl kleiner Mulden oder Lagunen 17 besitzt. An den Kragenbereich 16 schließt ein Rumpfbereich 18 an, der sich über den größten Teil der Länge des Implantatzylinders 11 erstreckt. Der Rumpfbereich 18 weist an seinem Umfang in axialer Richtung verlaufende rillenförmige Vertiefungen 20 auf, hierin auch Furchen genannt, die einander dicht benachbart sind. Die Vertiefungen 20 sind vorzugsweise mit einen konkaven Profil ausgestattet (Figur 2), das an seinen Rändern in den Umfang des Rumpfbereiches 18 ausläuft und mit jedem der beiden benachbarten Vertiefungen einen abgerundeten Kamm 22 bildet. Die rillenförmigen Vertiefungen haben eine Breite im Bereich zwischen 20 und 300 Mikrometern und eine Tiefe im Bereich von 10 bis 150 Mikrometern. Während der Einheilphase können sich in die rillenförmigen Vertiefungen die Osteone des das Implantat umgebenden Knochengewebes anlagern. Hierzu entspricht der Breitenbereich der Rillen den Abmessungen der Osteone.

Die gesamte Oberfläche des Rumpfbereichs 18 ist des weiteren mit einer Vielzahl kleiner Mulden oder Lagunen versehen, deren durchschnittlicher Durchmesser vorzugsweise in der Größenordnung von 3 Mikrometern und deren Tiefe vorzugsweise in der Größenordnung von 1 Mikrometer liegt und die das Einwachsen des Implantats unterstützen. Nach unten wird der Zylinder 11 durch einen abgerundeten Fußbereich 24 abgeschlossen, der an seiner Oberfläche ebenfalls eine Vielzahl kleiner Mulden oder Lagunen besitzt.

Die Figuren 3 bis 6 zeigen in vergrößerter Darstellung die Mulden oder Lagunen in den Bereichen 16, 18 und 24 des Zylinders 11. In Figur 3 ist ein Ausschnitt von der Oberfläche des Kragenbereichs 16 vergrößert dargestellt. Der Ausschnitt zeigt eine Vielzahl kleiner scharf begrenzter, kalottenförmiger Mulden 17, die dicht nebeneinander über die Oberfläche des Kragenbereichs 16 verteilt sind. Die Mulden 17 verstärken die Sperrwirkung des Kragenbereichs 16 gegen das Eindringen von Bakterien aus dem Rachenraum in den Aufnahmekanal des Implantats. Die Schnittdastellung von Figur 4 zeigt das kalottenförmige Profil der Mulden 17, die in noch zu beschreibender weise durch Säureätzung oder durch Anwendung der Lasertechnik hergestellt werden. Eine Oberflächenstruktur, wie sie die Figuren 3 und 4 zeigen, weist auch der Fußbereich 24 des Zylinders 11 auf. Die Figuren 5 und 6 zeigen die Anordnung entsprechender Mulden am Umfang des Rumpfbereiches 18. In Figur 5 ist ein Ausschnitt von der Oberfläche des Rumpfbereichs 18 vergrößert dargestellt. Der Ausschnitt zeigt eine Vielzahl kleiner scharf begrenzter, kalottenförmiger Mulden 23, die dicht nebeneinder über die Oberfläche der rillenförmigen Vertiefungen 20 und der Kämme 22 verteilt sind. Die Schnittdarstellung von Figur 6 zeigt das kalottenförmige Profil der Mulden 23.

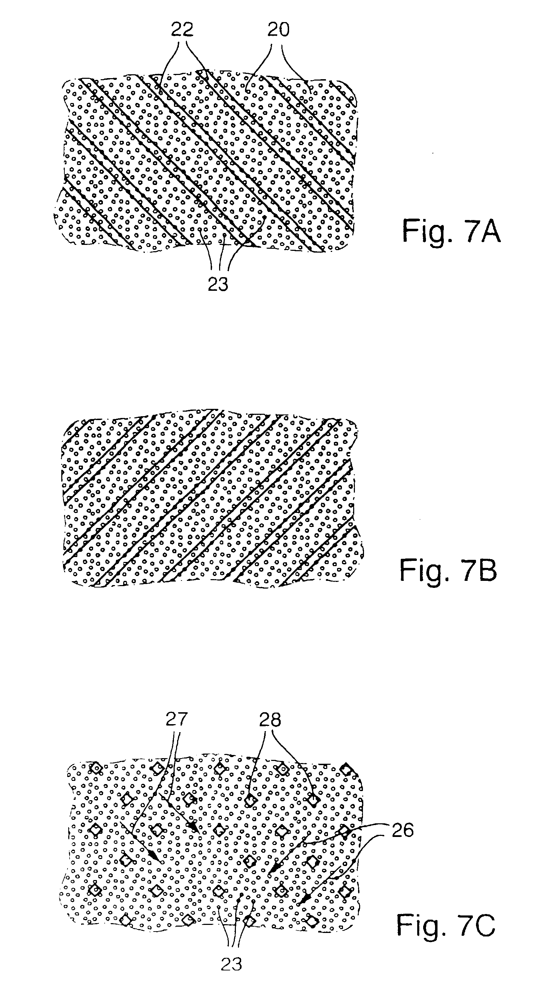

Abweichend von der in Figur 1 dargestellten Form können die rillenförmigen Vertiefungen 20 in einem spitzen Winkel zur Längsachse des Implantats verlaufen, beispielsweise in einem Winkel von 45°, so daß sich ein nach links oder nach rechts gewendelter Verlauf der Rillen ergibt, wie es die Figuren 7A und 7B in einer vergrößerten Draufsicht auf einen Ausschnitt des Rumpfbereichs eines entsprechend ausgebildeten Zahnimplantats zeigen. In Figur 7C ist ein kreuzweiser Verlauf der Rillen dargestellt. Bei dieser Ausführung sind erste Rillen vorgesehen, die in einer ersten Richtung 26, in Figur 7C nach links, in einem spitzen Winkel zur Längsachse des Implantatkörpers verlaufen, und es sind zweite Rillen vorgesehen, die in einer zweiten Richtung 27, d.h. nach rechts, in einem spitzen Winkel zur Längsachse des Implantatkörpers verlaufen und sich mit den ersten Rillen kreuzen, so daß eine Vielzahl noppenförmige, abgerundete Erhöhungen 28 entstehen, die sich gleichförmig über die Oberfläche des Rumpfbereichs 18 verteilen. Die in den Figuren 7A, 7B und 7C gezeigten Rillenstrukturen weisen vorzugsweise ein Profil auf, wie es in Figur 2 dargestellt ist, und bieten günstige Vorraussetzungen für die Anlagerung der Osteone während der Einheilphase, und sie bewirken eine zusätzliche Sicherung gegen axiale Verlagerung und Verdrehung des Implantats im eingewachsenen Zustand.

In den rillenförmigen Vertiefungen können zusätzlich kleine Mulden oder Lagunen 23 angeordnet sein, die gegenüber den Rillen klein sind und sich in einer hohen Dichte über die Oberfläche des Rumpfbereiches verteilen, wie es in den Figuren 5 und 6 dargestellt ist. Die Mulden oder Lagunen 23 unterstützen die Verankerung des Implantats im Kieferknochen, indem sie die Einlagerung einer hohen Anzahl von Osteozyten in die Oberfläche des Rumpfbereiches ermöglichen.

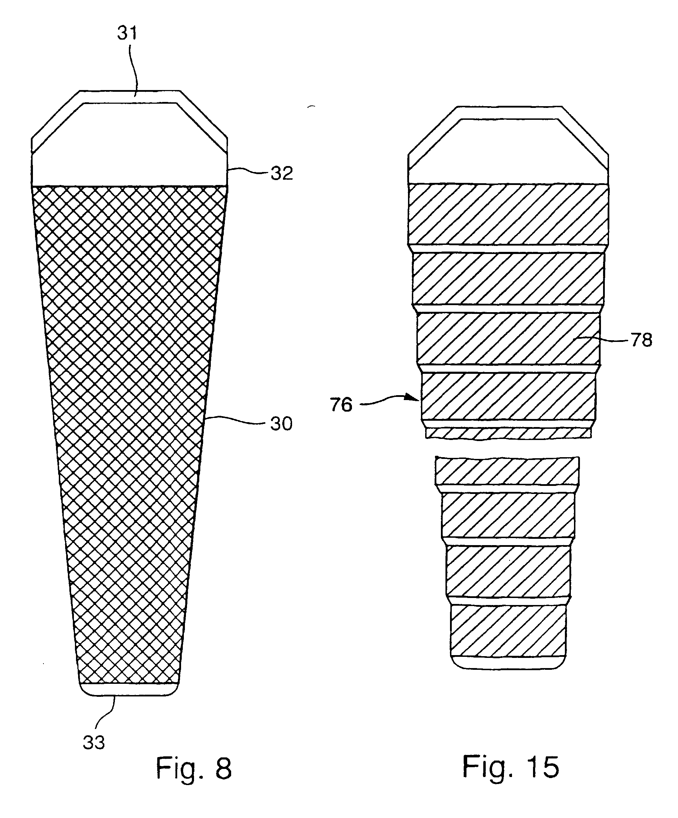

Die Figur 8 zeigt eine Abwandlung des beispielhaften Zahnimplantats nach Figur 1. Bei dieser Ausführungsform ist ein Rumpfbereich 30 kegelförmig ausgebildet und weist ein Oberflächenstruktur gemäß Figur 7C mit kreuzweise verlaufenden Rillen gemäß Figur 7C auf, die in Figur 8 lediglich schematisch dargestellt sind. Ein Kopfbereich 31 und ein Kragenbereich 32 des in Figur 8 gezeigten Implantatkörpers, der wie auch die Implantatkörper der nachfolgend beschriebenen Ausführungsbeispiele der Erfindung aus Titan hergestellt ist, entspricht den Bereichen 12 und 16 von Figur 1. Abweichend von Figur 1 kann das Implantat von Figur 8 an seinem Fußbereich 33 glatt poliert sein, wenn es in Verbindung mit einer Sinuslift-Operation zum Einsatz kommt.

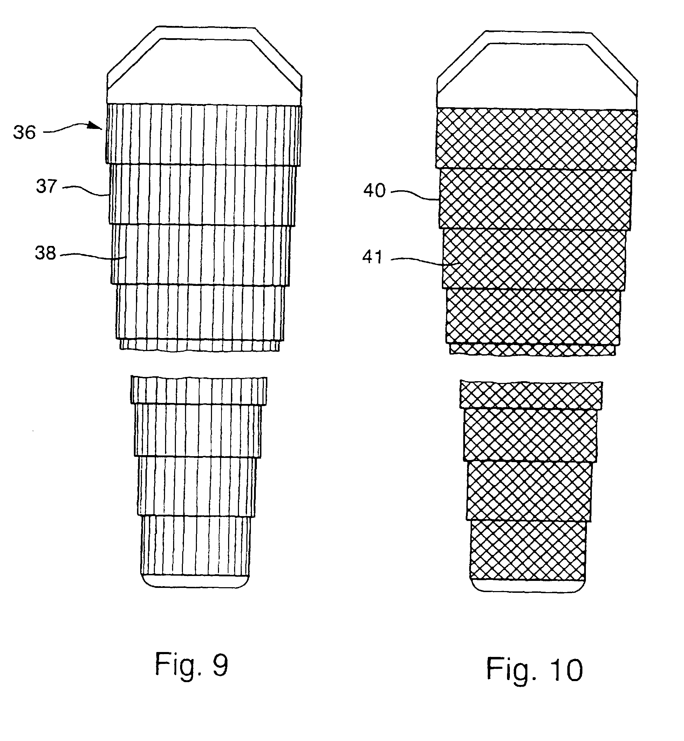

Die Figuren 9 und 10 zeigen Ausführungsformen des Zahnimplantats nach der Erfindung, bei denen sich der Rumpfbereich in Stufen nach unten verjüngt. Es können eine Vielzahl von Stufen vorgesehen sein, wobei die Stufenhöhe vorzugsweise in der Größenordnung von 20 bis 300 Mikrometern liegen kann. Der Umfangsbereich einer jeden Stufe ist mit einer Rillenstruktur gemäß Figur 7A, 7B oder 7C ausgestattet. Die Figur 9 zeigt ein Zahnimplantat mit einem Rumpfbereich 36, bei dem der Umfangsbereich einer jeden Stufe 37 in Richtung der Längsachse des Implantatkörpers verlaufende Rillen 38 aufweist, die vorzugsweise über alle Stufen 37 die gleiche Breite besitzen. Hierdurch ergibt sich eine unterschiedliche Anzahl Rillen 38 in jeder Stufe, und die Rillen einer jeden Stufe sind gegenüber den Rillen der vorausgehenden Stufe winkelversetzt. Im Kopfbereich, Kragenbereich und Fußbereich entspricht das Implantat von Figur 9 dem von Figur 8. Das Implantat gemäß Figur 10 unterscheidet sich vom Implantat gemäß Figur 9 dadurch, daß der Umfangsbereich einer jeden Stufe 40 mit einer Kreuzrillenstruktur 41 gemäß Figur 7C ausgestattet ist, die in Figur 10 schematisch dargestellt ist.

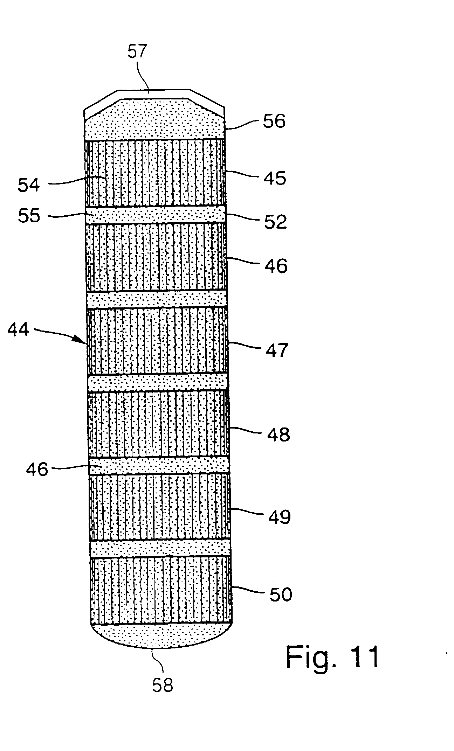

Weitere Ausführungsformen des Zahnimplantats gemäß der Erfindung sind in den Figuren 11 bis 17 dargestellt. Die Figur 11 zeigt einen aus Titan bestehenden Zylinder 44, der in eine Anzahl Abschnitte 45-50 unterteilt ist. Je zwei benachbarte der Abschnitte 45-50 sind durch ein schmales Band 52 getrennt, das als Barriere gegen von oben aus dem Rachenraum eindringende Infektionsherde dient und hierin auch als Barrierebereich bezeichnet wird. Jeder der Abschnitte 45-50 weist an seinem Umfang in axialer Richtung verlaufende rillenförmige Vertiefungen 54 auf, die einander dicht benachbart sind und vorzugsweise ein konkaves Profil der in Figur 2 dargestellten Art haben. Anstelle der axial verlaufenden Vertiefungen können die Abschnitte 45-50 auch schräg oder kreuzweise verlaufenden Rillen der in den Figuren 7A, 7B und 7C dargestellten Art aufweisen. Jeder der Trenn- oder Barrierebereiche 52 weist an seinem Umfang eine Vielzahl kleiner Mulden 55 der in den Figuren 3 und 4 dargestellten Art auf, welche die Barrierewirkung gegen Bakterien unterstützen. Ebenso sind die Abschnitte 45-50 nach Art der Figuren 5 und 6 mit Mulden versehen, die gegenüber den Abmessungen der Vertiefungen 54 klein sind. Auch ein Kragenbereich 56, der sich zwischen dem obersten Abschnitt 45 und einem polierten Kopfbereich 57 befindet, weist besagte Mulden auf. Nach unten wird der Zylinder 44 durch einen abgerundeten Fußbereich 58 abgeschlossen, der für besondere Anwendungen, wie insbesondere für die Verwendung für einem Sinuslift, hochglanzpoliert ist.

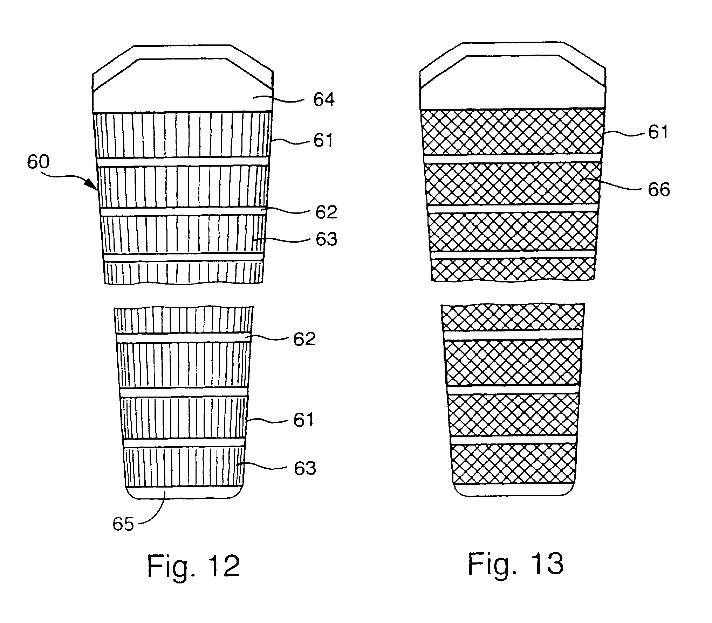

In den Figuren 12 und 13 sind entsprechende Ausführungen in Kegelform dargestellt. In Figur 12 ist der Rumpfbereich eines kegelförmigen Zahnimplantats 60 in Abschnitte 61 unterteilt, von denen je zwei durch einen Barrierebereich 62 getrennt sind. Die Abschnitte weisen in Längsrichtung des Kegels verlaufende Rillen 63 auf, die der Kegeloberfläche in Längsrichtung folgen. Die Rillen 63 sind vorzugsweise so angeordnet, daß sie von oben nach unten gesehen am Beginn eines jeden Abschnitts 61 die gleiche Breite besitzen und sich in ihrer Breite und Tiefe nach unten verjüngen. Die Oberflächen der Barrierebereiche 62 sind wie auch die Oberflächen eines Kragenbereichs 64 am oberen Ende des Implantats 60 und eines Fußbereichs 65 mit einer Vielzahl kleiner Mulden der in den Figuren 3 und 4 dargestellten Art versehen. Entsprechende Mulden sind auch im Bereich der Rillen 63 in der in den Figuren 3 und 4 dargestellten Art vorgesehen. Die Ausführungsform nach Figur 13 unterscheidet sich von der Ausführungsform nach Figur 12 dadurch, daß die Abschnitte 61 eine Rillenanordnung gemäß Figur 7C aufweisen, indem in jedem Abschnitt 61 sich kreuzende Rillen 66 nach links und rechts im spitzen Winkel zur Kegelachse verlaufen. In Figur 13 sind diese Rillen lediglich schematisch dargestellt.

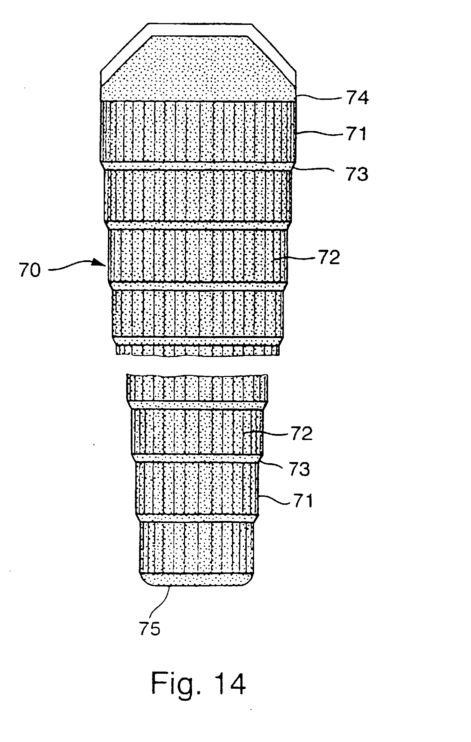

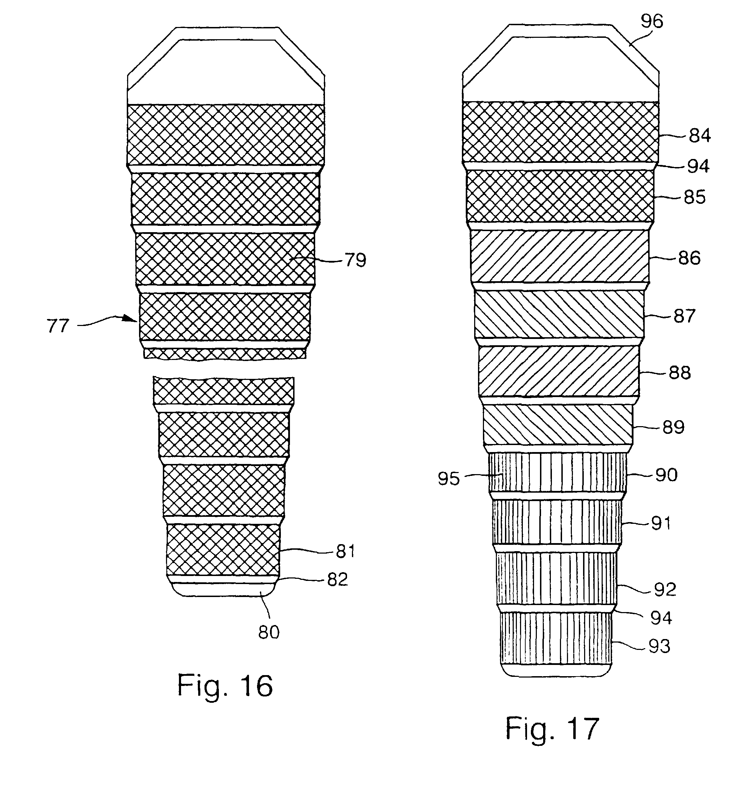

Die Figuren 14 bis 17 zeigen Ausführungsformen der Erfindung, bei denen sich der Rumpfbereich eines rotationssymmetrischen Implantatkörpers in Stufen nach unten verjüngt. Je zwei der Stufen sind durch einen kegligen Bereich getrennt. Der Umfangsbereich einer jeden Stufe ist mit einer Rillenstruktur gemäß Figur 7A, 7B oder 7C ausgestattet. Die Figur 14 stellt ein Zahnimplantat mit einem Rumpfbereich 70 dar, der in eine Vielzahl Stufen 71 unterteilt ist. Jede der Stufen 71 weist in Richtung der Längsachse des Implantatkörpers verlaufende Rillen 72 auf. Zwischen je zwei der Stufen 71 befindet sich ein kegelförmiger Übergangsbereich 73, der im Verhältnis zur Länge der Stufen 71 schmal ist und in der oben beschriebenen Weise als Barrierebereich wirkt. Die Rillen 72 besitzen vorzugsweise über alle Stufen 71 die gleiche Breite. Hierdurch ist die Zahl der Rillen 72 in jeder der Stufen 71 unterschiedlich, und die Rillen einer jeden Stufe sind gegenüber den Rillen der vorausgehenden Stufe winkelversetzt. Die Stufen 71, die Übergangsbereiche 73 und ein Kragenbereich 74 sowie ein Fußbereich 75 sind an ihren Oberflächen mit einer Vielzahl kleiner Mulden der in den Figuren 3 bis 6 dargestellten Art versehen. Die Figur 15 zeigt einen entsprechenden Implantatkörper 76, bei dem die Stufen 71 mit schräg verlaufenden Rillen 78 nach Art von Figur 7B ausgestattet ist, und Figur 16 zeigt in schematischer Darstellung einen entsprecheden Implantatkörper 77, der mit kreuzweise verlaufenden Rillen 79 nach Art von Figur 7C ausgestattet ist. Der Implantatkörper 77 nach Figur 16 weist außerdem einen polierten Fuß 80 auf, der mit der untersten Stufe 81 durch einen ebenfalls polierten kegligen Übergangsbereich 82 verbunden ist.

In Figur 17 ist eine Ausführungsform des erfindungsgemäßen Zahnimplantats nach Art der Figur 14 dargestellt, bei dem wenigstens ein Teil der Stufen unterschiedliche Rillenstrukturen aufweisen. Der Rumpfbereich des Implantats nach Figur 17 ist in zehn Stufen 84-93 unterteilt, die durch Übergangsbereiche 94 miteinander verbunden sind. Die beiden obersten Stufen 84 und 85 sind an ihrem Umfang mit kreuzweise verlaufenden Rillen nach Art von Figur 7C ausgestattet. Von den nach unten folgenden Stufen 86 und 87 weist die Stufe 86 in einem spitzen Winkel zur Längsachse des Implantats nach links verlaufende Rillen und die Stufe 87 in einem spitzen Winkel zu Längsachse nach rechts verlaufende Rillen auf. Die nächsten Stufen 88 und 89 entsprechen in ihrer Oberflächenstruktur den Stufen 86 und 87, während alle weiteren Stufen 90 bis 93 in axialer Richtung verlaufende Rillen 95 aufweisen. Auch die Breite der Rillen kann in den einzelnen Stufen unterschiedlich sein. So können die Stufen 90 bis 93, die von einem porösem Knochengewebe umgeben sind, eine Breite aufweisen, die größer ist als die Breite der darüber befindlichen Stufen. Die gesamte Oberfläche des Implantats nach Figur 17 ist mit Mulden oder Lagunen der in Figur 3 bis 6 dargestellten Art bedeckt mit Ausnahme eines Kopfbereichs 96, der eine glatte und polierte Oberfläche besitzt.

Die über die Stufen 84-93 wechselnde Oberflächenstruktur berücksichtigt einerseits die unterschiedliche Dichte des Knochens, der das Implantat über dessen Länge umgibt. So bilden insbesondere die beiden oberen Stufen 84, 85 mit ihren kreuzweise verlaufenden Rillen eine gute Verankerung des Implantats innerhalb der Compakta und dem angrenzenden Bereich, während die Oberflächen der folgenden Stufen dem nach unten zunehmend proröseren Knochengewebe angepaßt werden können. Die Kombination unterschiedlicher Oberflächenstrukturen über den Rumpfbereich des Implantats unterstützt auch das Ziel, das Implantat während der Einheilphase und im eingeheilten Zustand gegen axiale Verlagerungen und Verdrehungen zu sichern und den Druck besser in den Knochen einzuleiten.

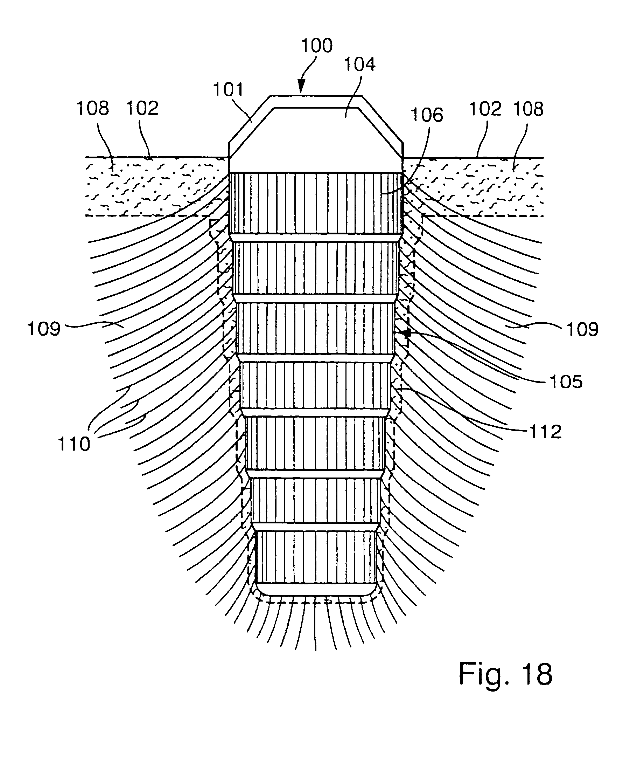

Die Figur 18 zeigt den Sitz eines erfindungsgemäßen Zahnimplantats 100 im Kieferknochen nach Abschluß der Einheilphase. Das Implantat 100 entspricht der in Verbindung mit Figur 14 beschriebenen Art. Das Implantat wird so tief in den Kieferknochen eingesetzt, daß sein Kopfbereich 101 oberhalb des oberen Randes des Kieferknochens 102 liegt. Der Kragenbereich 104 und ein Teil des Rumpfbereichs 105 bis zum unteren Drittel der ersten oder obersten Stufe 106 befinden sich im Bereich natürlichen Compakta 108, die sich unter dem Rand des Kieferknochens befindet und an die sich nach unten die Spongiosa 109 des Kieferknochens anschließt. Die rillenförmige Oberfläche des Implantats bewirkt, daß sich die Osteone 110 des Knochens in die Rillen am Umfang der Stufen 106 einlagern. Auf diese Weise kommt es zu einer innigen Verbindung zwischen Knochen und Implantat. Durch die Rillenstruktur wird es den Osteonen ermöglicht, direkt an das Implantat heranzuwachsen. Dies verbessert die Druckübertragung auf die anatomisch-histologischen Knochenbestandteile, insbesondere auf die Osteone, und führt im Bereich der Spongiosa zur Bildung einer druckinduzierten Ausbildung einer Compakta 112 um das Implantat. Durch diese biomechanische Integration wird die Einheilphase des Implantats verkürzt und aufgrund der geringeren Knochenresorbtion dessen Standzeit im Knochen verlängert.

Ein bevorzugtes Verfahren zur Herstellung des Zahnimplantats gemäß der Erfindung besteht darin, daß nach einem bekannten Verfahren wie z.B. dem Druckgußverfahren der Implantatkörper in der gewünschten Form als Zylinder, Kegel oder abgestufter Kegel jeweils mit einer glatten Oberfläche hergestellt wird. Danach wird der Implantatkörper einer Materialabtragungsoperation unterzogen, durch die eine Vielzahl von im wesentlichen in Richtung der Längsachse verlaufender rillenförmiger Vertiefungen in die Oberfläche des Implantatkörpers eingebracht werden. Die rillenförmigen Vertiefungen 20 können parallel zur Längsachse des Implantatkörpers oder zu seiner Oberfläche in Falle einer Kegelform verlaufen oder in einem spitzen Winkel dazu in der in den Figuren 7A, 7B oder 7C dargestellten Art. Das Profil und die Abmessungen der Vertiefungen entsprechen dem Profil und den Abmessungen, wie sie oben insbesondere in Verbindung mit den Figuren 2, 5 und 6 beschrieben wurden. Die Materialabtragungsoperation kann vorzugsweise durch einen digital gesteuerten Laserstrahl ausgeführt werden. Anstelle des Laserstrahls kann auch ein fokussierter Ionenstrahl Anwendung finden. Alternativ kann die Materialabtragungsoperation durch eine feinmechanische Fräßmaschine ausgeführt werden.

In einem weiteren Schritt werden die Mulden oder Lagunen 17 in die Oberfläche des Implantatkörpers eingebracht. Dieser Schritt kann aus einer Säureätzung bestehen, wobei der Kopfbereich 12, 31, 57, 96 oder 101 und in bestimmten Fällen auch der Fußbereich 33 oder 80 jeweils mit einer säureresistenten Schicht abgedeckt wird. Zur Einbringung der Mulden oder Lagunen 17 kann aber auch ein digital gesteuerter Laserstrahl verwendet werden. Dies hat den Vorzug, daß zur Herstellung der rillenförmigen Vertiefungen 20 und zur Herstellung der Mulden oder Lagunen 17 die gleichen Halte- und Vorschubvorrichtungen, die gleiche Werkzeugart und das gleiche Materialabtragungsverfahren Anwendung finden können.

Anstatt einer direkten Anwendung der Materialabtragungsoperationen mittels Laserstrahl oder Ionenstrahl auf den Implantatkörper kann diese Bearbeitung auch auf eine Druckgußform angewendet werden, die zur Herstellung des Implantats unter Anwendung eines bekannten Druckgußverfahrens dient. In diesem Falle wird ein Negativ der beschriebenen Oberflächenstrukturen in eine geteilte Druckgußform eingearbeitet, die entsprechend der herzustellenden Form des Implantats als Zylinder, Kegel oder abgestufter Kegel jeweils mit einer glatten Oberfläche ausgebildet ist.

## Patentansprüche

1. Zahnimplantat mit einem rotationssymmetrischen Implantatkörper (11, 30), der eine gerauhte Oberfläche mit einer Vielzahl rillenförmiger Vertiefungen aufweist, wobei die Oberfläche des Implantatkörpers in Richtung seiner Längsachse in eine Anzahl Abschnitte unterteilt ist, dadurch gekennzeichnet, daß diese Abschnite durch radial verlaufende Bänder (52, 62) voneinander getrennt sind, und daß jeder der Abschnitte (45-50, 61) an seinem Umfang eine Vielzahl in Richtung der Längsachse des Implantatkörpers oder in einem spitzen Winkel zu dieser verlaufende rillenförmige Vertiefungen (20, 54, 63) aufweist, deren Größenordnung der Größenordnung der Osteone des Kieferknochengewebes entspricht, die sich an die rillenförmigen Vertiefungen anlagern.

2. Zahnimplantat nach Anspruch 1, dadurch gekennzeichnet, daß die Abschnitte abgestufte Durchmesser aufweisen, die zur Bildung einer Kegelform zum unteren Endes des Implantatkörpers stufenförmig abnehmen (37, 40, 71).

3. Zahnimplantat nach Anspruch 1 und 2, dadurch gekennzeichnet, daß die radial verlaufenden Bänder (62) durch die Stufen (37, 40, 71) zwischen den Abschnitten gebildet werden.

4. Zahnimplantat nach Anspruch 1 oder 2, dadurch gekennzeichnet, daß am Umfang der Abschnitte (40, 61) erste Rillen vorgesehen sind, die in einer ersten Richtung in einem spitzen Winkel zur Längsachse des Implantatkörpers verlaufen, und daß am Umfang der Abschnitte zweite Rillen vorgesehen sind, die in einer zweiten Richtung in einem spitzen Winkel zur Längsachse des Implantatkörpers verlaufen und sich mit den ersten Rillen kreuzen (41, 66, 79).

5. Zahnimplantat nach Anspruch 1, dadurch gekennzeichnet, daß die rillenförmigen Vertiefungen (20) dicht benachbart sind und ein konkaves Profil aufweisen, das an seinen Rändern in den Umfang der Abschnitte (37, 40, 45-50, 61, 71) ausläuft.

6. Zahnimplantat nach Anspruch 1, dadurch gekennzeichnet, daß in den rillenförmigen Vertiefungen (20) in Bezug auf diese klein ausgebildete muldenförmige Vertiefungen (17) räumlich verteilt angeordnet sind.

7. Zahnimplantat nach Anspruch 1, dadurch gekennzeichnet, daß die Breite der rillenförmigen Vertiefungen (20) im Bereich von 20 bis 300 Mikrometer liegt.

8. Zahnimplantat nach Anspruch 1, dadurch gekennzeichnet, daß die Tiefe der rillenförmigen Vertiefungen (20) in der Größenordnung von 10 bis 150 Mikrometern liegt.

9. Zahnimplantat nach den Ansprüchen 1 und 2, dadurch gekennzeichnet, daß sich der Durchmesser der Abschnitte jeweils im Bereich der radial verlaufenden Bänder (73) verjüngt.

10. Zahnimplantat nach den Ansprüchen 1 und 2, dadurch gekennzeichnet, daß die rillenförmigen Vertiefungen in den Abschnitten (37, 40, 61 oder 71) über die Länge des Implantatkörpers im wesentlichen die gleichen Abmessungen aufweisen.

11. Zahnimplantat nach den Ansprüchen 1 und 2, dadurch gekennzeichnet, daß wenigstens ein Teil der Abschnitte (84-90) über die Länge des Implantatkörpers unterschiedliche Rillenstrukturen aufweisen.

12. Zahnimplantat nach den Ansprüchen 1 und 2, dadurch gekennzeichnet, daß die rillenförmigen Vertiefungen in den Abschnitten (84-90) über die Länge des Implantatkörpers unterschiedliche Abmessungen aufweisen und daß die Abmessungen an die über die Länge des Implantatkörpers unterschiedliche Dichte des Knochengewebes angepaßt sind.

13. Zahnimplantat nach Anspruch 12, dadurch gekennzeichnet, daß die rillenförmigen Vertiefungen über die Länge des Implantatkörpers in einem Teil der Abschnitte (90-93) eine Breite aufweisen, die von der Breite der rillenförmigen Vertiefungen in darüber befindlichen Abschnitten (84-89) im Bereich von 10 bis 150 Mikrometern abweicht.

14. Zahnimplantat nach wenigstens einem der Ansprüche 9 bis 13, dadurch gekennzeichnet, daß die rillenförmigen Vertiefungen (38, 41, 63, 66, 78, 79) dicht benachbart sind und ein konkaves Profil aufweisen, das an seinen Rändern in den Umfang des jeweiligen Abschnitts (37, 40, 61, 71 oder 84-90) ausläuft.

15. Zahnimplantat nach wenigstens einem der Ansprüche 9 bis 14, dadurch gekennzeichnet, daß die Breite der rillenförmigen Vertiefungen (38, 41, 63, 66, 78, 79) im Bereich von 20 bis 300 Mikrometern liegt.

16. Zahnimplantat nach wenigstens einem der Ansprüche 9 bis 15, dadurch gekennzeichnet, daß die Tiefe der rillenförmigen Vertiefungen der Größenordnung von 10 bis 150 Mikrometern liegt.

17. Zahnimplantat nach wenigstens einem der Ansprüche 9 bis 16, dadurch gekennzeichnet, daß in den rillenförmigen Vertiefungen (20, 38, 41, 63, 66, 78, 79) zusätzlich eine Vielzahl von in Bezug auf diese klein ausgebildete Mulden (17) räumlich verteilt angeordnet sind.

18. Zahnimplantat nach Anspruch 17, dadurch gekennzeichnet, daß die Breite der Mulden in der Größenordnung von drei Mikrometern und ihre Tiefe in der Größenordnung von einem Mikrometer liegt.

19. Zahnimplantat nach Ansprüche 1, dadurch gekennzeichnet, daß der Implantatkörper zwischen den rillenförmigen Vertiefungen und einem Implantatkopf einen den Umfang des Implantatkörpers überdeckenden Kragenbereich (64, 74) aufweist, an dessen Umfang eine Vielzahl kleiner räumlich verteilter Mulden (17) angeordnet sind, und daß der Implantatkörper (77) an seinem unteren Ende einen glatt polierten Fußbereich (80) aufweist.

20. Zahnimplantat nach Anspruch 1 und 2, dadurch gekennzeichnet, daß die Abstufung der Durchmesser der Abschnitte in der Größenordnung von 20 bis 300 Mikrometern liegt.

21. Verfahren zur Herstellung eines Zahnimplantats mit einem vorgeformten rotationssymmetrischen Implantatkörper (30, 44), der eine gerauhte Oberfläche aufweist, in die durch eine Materialabtragungsoperation eine Vielzahl wobei rillenförmiger Vertiefungen eingebracht werden, die Oberfläche des Implantatkörpers in Richtung seiner Längsachse in eine Anzahl Abschnitte (45-50, 61) unterteilt ist, dadurch gekennzeichnet, daß diese Abschnitte durch radial verlaufende Bänder (52, 62) voneinander getrennt sind, und daß in jeden der Abschnitte an dessen Umfang eine Vielzahl in Richtung der Längsachse des Implantatkörpers oder in einem spitzen Winkel zu dieser verlaufende rillenförmige Vertiefungen (20, 54, 63) durch eine Materialabtragungsoperation eingebracht werden, und daß die Größenordnung der rillenförmigen Vertiefungen der Größenordnung der Osteone des Kiefernknochengewebes entspricht, die sich an die rillenförmigen Vertiefungen anlagern.

22. Verfahren nach Anspruch 21, dadurch gekennzeichnet, daß die Abschnitte in abgestuften Durchmessern vorgeformt werden, die zur Bildung einer Kegelform zum unteren Ende des Implantatkörpers stufenförmig abnehmen (37, 40, 71).

23. Verfahren nach Anspruch 21, dadurch gekennzeichnet, daß innerhalb der radial verlaufenden Bänder (52, 62) besagte Materialabtragungsoperation zur Herstellung der rillenförmigen Vertiefungen (20, 54, 63) unterbrochen wird.

24. Verfahren nach Anspruch 22, daß die radial verlaufenden Bänder durch die Stufen (37, 40, 71) zwischen den Abschnitten gebildet werden.

25. Verfahren nach Anspruch 22, dadurch gekennzeichnet, daß die Abstufung der Durchmesser in der Größenordnung von 20 bis 300 Mikrometern liegt.

26. Verfahren nach Anspruch 21, dadurch gekennzeichnet, daß die rillenförmigen Vertiefungen (20) dicht benachbart angeordnet werden und ein konkaves Profil aufweisen, das an seinen Rändern in den Umfang der Abschnitte (37, 40, 45-50, 61, 71) ausläuft.

27. Verfahren nach Anspruch 21, dadurch gekennzeichnet, daß die Breite der rillenförmigen Vertiefungen (20) im Bereich von 20 bis 300 Mikrometern liegt und daß ihre Tiefe in der Größenordnung von 10 bis 150 Mikrometern liegt.

28. Verfahren nach Anspruch 21, dadurch gekennzeichnet, daß in die rillenförmigen Vertiefungen (20) der Abschnitte durch eine weitere Materialabtragungsoperation eine Vielzahl von in Bezug auf die rillenförmigen Vertiefungen klein ausgebildeten muldenförmigen Vertiefungen (17) räumlich verteilt eingebracht werden.

29. Verfahren nach den Ansprüchen 21 und 23, dadurch gekennzeichnet, daß ein rotationssymmetrischer Implantatkörper (36, 70) mit Stufen (37, 71, 85-93) unterschiedlichen Durchmessers durch Anwendung eines Sinterverfahrens vorgeformt wird, und daß in die Oberfläche einer jeden der Stufen durch eine Materialabtragungsoperation eine Vielzahl in Richtung der Längsachse des Implantatkörpers oder in einem spitzen Winkel zu dieser verlaufende rillenförmige Vertiefungen (38, 72, 95) eingebracht werden.

30. Verfahren nach Anspruch 29, dadurch gekennzeichnet, daß die rillenförmigen Vertiefungen (38, 72, 95) in den Stufen (37, 71, 85-93) über die Länge des Implantatkörpers im wesentlichen die gleichen Abmessungen aufweisen.

31. Verfahren Anspruch 29, dadurch gekennzeichnet, daß durch die Materialabtragungsoperation in wenigstens einen Teil der Stufen (84-90) über die Länge des Implantatkörpers unterschiedliche Rillenstrukturen und/oder rillenförmige Vertiefungen unterschiedlicher Größe eingebracht werden.

32. Verfahren nach den Ansprüche 22 und 24, dadurch gekennzeichnet, daß in die Bänder (62, 73) durch eine Materialabtragungsoperation eine Vielzahl kleiner Mulden (17) räumlich verteilt eingebracht werden.

33. Verfahren nach einem der Ansprüche 21 bis 31, dadurch gekennzeichnet, daß oberhalb der Abschnitte ein den Umfang des Implantatkörpers überdeckender Kragenbereich (64, 74) angeordnet wird, in den durch eine Materialabtragungsoperation eine Vielzahl kleiner Mulden (17) räumlich verteilt eingearbeitet werden.

34. Verfahren nach einem der Ansprüche 21 bis 33, dadurch gekennzeichnet, daß die Materialabtragungsoperationen durch einen Laserstrahl erfolgen.

35. Verfahren nach einem der Ansprüche 21 bis 34, dadurch gekennzeichnet, daß die Materialabtragungsoperationen durch eine Feinfräßmaschine erfolgen.

36. Verfahren nach einem der Ansprüche 26 bis 34, dadurch gekennzeichnet, daß die muldenförmigen Vertiefungen (17) durch Ätzen hergestellt werden.

37. Verfahren nach Anspruch 36, dadurch gekennzeichnet, daß die Tiefe der Mulden (17) in der Größenordnung von drei Mikrometern und ihre Tiefe in der Größenordnung von einem Mikrometer liegt.

38. Verfahren nach Anspruch 36, dadurch gekennzeichnet, daß der Ätzvorgang gleichzeitig auf die rillenförmigen Vertiefungen (20, 38, 63, 72, 78, 95) und auf den den Umfang des Implantatkörpers überdeckenden Kragenbereich (64, 74) angewendet wird.

39. Verfahren nach Anspruch 36, dadurch gekennzeichnet, daß der Ätzvorgang zugleich auf die rillenförmigen Vertiefungen (20, 38, 63, 72, 78, 95) und auf die radial verlaufenden Bänder (62, 73) angewendet wird.

40. Verfahren nach einem der Ansprüche 21 bis 39, dadurch gekennzeichnet, daß die Materialabtragungsoperationen auf eine geteilte Druckgußform angewendet werden, die zur Herstellung des Implantats dient, daß die geteilte Druckgußform entsprechend der herzustellenden Form des Implantats als Zylinder, Kegel oder abgestufter Kegel jeweils mit einer glatten Oberfläche ausgebildet wird, und daß ein Negativ der besagten Oberflächenstrukturen in die Druckgußform eingearbeitet wird.

## Claims

1. A tooth implant with a rotationally symmetrical implant body (11, 30) that has a rough surface with a plurality of groove-shaped recesses (20), the surface of the implant body is divided in the direction of its longitudinal axis into a number of sections,
characterized in that,
the sections are separated from each other by radial extending bands (52, 62) and that each of the sections (45-50, 61) comprises a plurality of groove-shaped recesses (20, 54, 63) at its periphery which are oriented in the direction of the longitudinal axis of the implant body or at a sharp angle to it, the groove-shaped recesses having an order of size that corresponds to the order of size of the osteons in the jawbone tissue which attach to the groove-shaped recesses.

2. The tooth implant according to claim 1, characterized in that the sections comprise stepped diameters which are decreasing toward the bottom end of the implant body to form a cone shape (37, 40, 71).

3. The tooth implant according to claim 1 and 2,
characterized in that the radial extending bands (62) are formed by the steps (37, 40, 71) between the sections.

4. The tooth implant according to claim 1 or 2, characterized in that the periphery of the sections (40, 60) comprises a first set of grooves which are oriented in a first direction at a sharp angle to the longitudinal axis of the implant body, and a second set of grooves which are oriented in a second direction at a sharp angle to the longitudinal axis of the implant body and cross the first grooves (41, 66, 79).

5. The tooth implant according to claims 1, characterized in that the groove-shaped recesses (20) are closely adjacent to each other and have a concave profile whose edges end in the periphery of the sections (37, 40, 45-50, 61, 71).

6. The tooth implant according to claim 1, characterized in that a plurality of trough-shaped recesses (17) are spatially distributed in the groove-shaped recesses (20) which trough-shaped recesses (17) are small in comparison with the groove-shaped recesses.

7. The tooth implant according to claim 1, characterized in that the width of the groove-shaped recesses (20) is in the range of 20 to 300 micrometers.

8. The tooth implant according to claim 1, characterized in that the depth of the groove-shaped recesses (20) is in the range of 10 to 150 micrometers.

9. The tooth implant according to claims 1 and 2, characterized in that the diameters of the sections are reduced in the area of the radial extending bands (73).

10. The tooth implant according to claims 1 and 2,
characterized in that the groove-shaped recesses (54) in the sections (37, 40, 61 or 71) have essentially the same dimensions over the length of the implant body.

11. The tooth implant according to claim 1 and 2,
characterized in that at least a part of the sections (84-90) have different groove structures over the length of the implant body.

12. The tooth implant according to claim 1 and 2,
characterized in that the groove-shaped recesses of the sections (84-90) have different dimensions over the length of the implant body, and that the dimensions are adapted to different density of the bone tissue over the length of the implant body.

13. The tooth implant according to claim 12, characterized in that over the length of the implant body the groove-shaped recesses comprise in a part of the sections (90-93) a width which deviates from the width in sections (84-89) above them in a range of 10 to 150 micrometers.

14. The tooth implant according to at least one of claims 9 to 13, characterized in that the groove-shaped recesses (38, 41, 63, 66, 78, 79) are closely adjacent to each other and have a concave profile whose edges end in the periphery of the sections (37, 40, 61, 71 or 84-90).

15. The tooth implant according to at least one of claims 9 to 14, characterized in that the width of the groove-shaped recesses (38, 41, 63, 66, 78, 79) is in the range of 20 to 300 micrometers.

16. The tooth implant according to at least one of claims 9 to 14, characterized in that the depth of the groove-shaped recesses is in the range of 10 to 150 micrometers.

17. The tooth implant according to at least one of claims 9 to 16, characterized in that the groove-shaped recesses (20, 38, 41, 63, 66, 78, 79) additionally comprises a plurality of small troughs (17) which are small in comparison with the groove-shaped recesses and which are distributed over the groove-shaped recesses.

18. The tooth implant according to claim 17, characterized in that the width of the troughs (17) is in the range of three micrometers and their depth is in the range of one micrometer.

19. The tooth implant according to claim 1, characterized in that the implant body comprises a collar area (64, 74) which is arranged between the groove-shaped recesses and an implant head and which covers the periphery of the implant body, that the collar area comprises at its periphery a plurality of small spatially distributed troughs (17), and that the implant body (77) comprises a smoothly polished foot area (80) at its bottom end.

20. The tooth implant according to claim 1 and 2,
characterized in that the diameter graduation of the sections is in the range of 20 to 300 micrometers.

21. A method of manufacture for a tooth implant with a preshaped rotationally symmetrical implant body (30, 44) having a rough surface on which a plurality of groove-shaped recesses are implemented by a material removing operation, the surface of the implant body is divided in the direction of its longitudinal axis into a number of sections (45-50, 61),
characterized in that
the sections are separated from each other by radial extending bands (52, 62), and that by a material removing operation in each of the sections (45-50, 61) at its periphery a plurality of groove-shaped recesses (20, 54, 63) are produced which extend in the direction of the longitudinal axis of the implant body or at a sharp angle to it, and that the groove-shaped recesses having an order of size that corresponds to the order of size of the osteons in the jawbone tissue which attach to the groove-shaped recesses.

22. The method according to claim 21, characterized in that a the sections are preformed in stepped diameters which are decreasing toward the bottom end of the implant body to form a cone shape (37, 40, 71).

23. The method according to claim 21, characterized in that within the radial extending bands (52, 62) said material removing operation for producing the groove-shaped recesses (20, 54, 63) is interrupted.

24. The method according to claim 22, characterized in that the radial extending bands are formed by the steps (37, 40, 71) between the sections.

25. The method according to claim 22, characterized in that the diameters graduation is in the range of 20 to 300 micrometers.

26. The method according to claim 21, characterized in that the groove-shaped recesses (20) are closely adjacent to each other and have a concave profile the edges of which end in the periphery of the sections (37, 40, 45-50, 61, 71).

27. The method according to claim 21, characterized in that the width of the groove-shaped recesses (20) is in the range of 20 to 300 micrometers, and that the depth of the groove-shaped recesses (20) is in the range of 10 to 150 micrometers.

28. The method according to claim 21, characterized in that a plurality of trough-shaped recesses (17) are implemented in the groove-shaped recesses (20) by an additional material removing operation, the trough-shaped recesses are small in comparison with the groove-shaped recesses and are spatially distributed over the groove-shaped recesses.

29. The method according to claim 21 and 23, characterized in that a rotationally symmetrical implant body (36, 70) with steps (37, 71, 85-93) of different diameters is preformed by using a sintering process, and that in the surface of each of the steps a plurality of groove-shaped recesses (38, 72, 95) are implemented by a material removing operation, the groove-shaped recesses which extend in the direction of the longitudinal axis of the implant body or at a sharp angle to this axis.

30. The method according to claim 29, characterized in that the groove-shaped recesses (38, 72, 95) in the steps (37, 71, 85-90) have essentially the same dimensions over the length of the implant body.

31. The method according to claim 29, characterized in that over the length of the implant body in at least a part of the steps (84-90) different groove structures and/or groove-shaped recesses of different size are produced by the material removing operation.

32. The method according to the claims 22 and 24, characterized in that a plurality of spatially distributed small troughs are implemented in the bands (62, 73) by a material removing operation.

33. The method according to one of the claims 21 to 31, characterized in that a collar area (64, 74) is arranged above the sections which covers the perimeter of the implant body, and a plurality of spatially distributed small troughs (71) are implemented in the collar area by a material removing operation.

34. The method according to one of claims 21 to 33,
characterized in that the material removing operation is carried out by a laser beam.

35. The method according to one of claims 21 to 34,
characterized in that the material removing operation is carried out by a precision milling machine.

36. The method according to one of claims 21 to 34,
characterized in that the trough-shaped recesses (17) are produced by etching.

37. The method according to claim 36, characterized in that the width of the troughs (17) is in the range of three micrometers and their depth is in the range of one micrometer.

38. The method according to claim 36, characterized in that the etching operation is simultaneously applied to the groove-shaped recesses (20, 38, 63, 72, 95) and to the collar area (64, 74) the perimeter of which covers the implant body.

39. The method according to claim 36, characterized in that the etching operation is simultaneously applied to the groove-shaped recesses (20, 38, 63, 72, 95) and to the radial extending bands (62, 73).

40. The method according to one of claim 21 to 39,
characterized in that the material removing operation is applied to a divided diecasting mold that is used to produce the implant, that the divided diecasting mold is formed as a cylinder, cone or stepped cone with a smooth surface corresponding to the shape of the implant to be produced, and that a negative representation of the said surface structures is implemented in the diecasting mold.

## Revendications

1. Implant dentaire ayant un corps d'implant (11, 30) présentant une symétrie de rotation qui a une surface rugueuse avec une pluralité d'évidements en forme de gorges (20), la surface du corps d'implant étant divisée dans la direction de son axe longitudinal en un certain nombre de sections,
caractérisé en ce que
les sections sont séparées les unes des autres par des bandes radiales étendues (52, 62) et en ce que chacune des sections (45-50, 61) comprend une pluralité d'évidements en forme de gorges (20, 54, 63) à sa périphérie qui sont orientées dans la direction de l'axe longitudinal du corps d'implant ou selon un angle aigu avec lui, les évidements en forme de gorges ayant un ordre de taille qui correspond à l'ordre de taille des ostéones dans le tissu du maxillaire qui sont attachés aux évidements en forme de gorges.

2. Implant dentaire selon la revendication 1, caractérisé en ce que les sections ont des diamètres qui sont dégressifs par pas successifs en allant vers le fond du corps d'implant pour former un cône.

3. Implant dentaire selon la revendication 1 ou 2, caractérisé en ce que les bandes radiales étendues (62) sont formées par les pas (37, 40, 71) entre les sections.

4. Implant dentaire selon la revendication 1 ou 2, caractérisé en ce que la périphérie des sections (40, 60) comprend un premier ensemble de gorges qui sont orientées dans une première direction selon un angle aigu avec l'axe longitudinal du corps d'implant, et un second ensemble de gorges qui sont orientées dans une seconde direction selon un angle aigu avec l'axe longitudinal du corps d'implant et croisent les premières gorges (41, 66, 79).

5. Implant dentaire selon la revendication 1, caractérisé en ce que les évidements en forme de gorges (20) sont étroitement adjacents entre eux et ont un profil concave dont les bords se terminent à la périphérie des sections (37, 40, 45-50, 61, 71).

6. Implant dentaire selon la revendication 1, caractérisé en ce que une pluralité d'évidements en forme de creux (17) sont répartis spatialement dans les évidements en forme de gorges (20), lesquels évidements en forme de creux sont petits en comparaison des évidements en forme de gorges.

7. Implant dentaire selon la revendication 1, caractérisé en ce que la largeur des évidements en forme de gorges (20) est comprise dans l'intervalle 20 à 300 microns.

8. Implant dentaire selon la revendication 1, caractérisé en ce que la profondeur des évidements en forme de gorges (20) est comprise dans l'intervalle 10 à 150 microns.

9. Implant dentaire selon les revendications 1 et 2, caractérisé en ce que les diamètres des sections sont réduits dans la zone des bandes radiales étendues (73).

10. Implant dentaire selon les revendications 1 et 2, caractérisé en ce que les évidements en forme de gorges (54) dans les sections (37, 40, 61, ou 71) ont essentiellement les mêmes dimensions sur toute la longueur du corps d'implant.

11. Implant dentaire selon les revendications 1 et 2, caractérisé en ce que au moins une partie des sections (84-90) ont des structures de gorges différentes sur toute la longueur du corps d'implant.

12. Implant dentaire selon les revendications 1 et 2, caractérisé en ce que les évidements en forme de gorges des sections (84-90) ont des dimensions différentes sur toute la longueur du corps d'implant, et en ce que les dimensions sont adaptées à la densité différente du tissu osseux sur toute la longueur du corps d'implant.

13. Implant dentaire selon la revendications 12, caractérisé en ce que, sur toute la longueur du corps d'implant, les évidements en forme de gorges comportent dans une partie des sections (90-93) une largeur qui est supérieure à la largeur des sections (84-89) d'une valeur comprise dans un intervalle de 10 à 150 microns.

14. Implant dentaire selon au moins une des revendications 9 à 13, caractérisé en ce que les évidements en forme de gorges (38, 41, 63, 66, 78, 79) sont étroitement adjacents entre eux et ont un profil concave dont les bords se terminent à la périphérie des sections (37, 40, 61, 71 ou 84-90).

15. Implant dentaire selon au moins une des revendications 9 à 14, caractérisé en ce que la largeur des évidements en forme de gorges (38, 41, 63, 66, 78, 79) est comprise dans l'intervalle 20 à 300 microns.

16. Implant dentaire selon au moins une des revendications 9 à 14, caractérisé en ce que la profondeur des évidements en forme de gorges est comprise dans l'intervalle 10 à 150 microns.

17. Implant dentaire selon au moins une des revendications 9 à 16, caractérisé en ce que les évidements en forme de gorges (38, 41, 63, 66, 78, 79) comportent en outre une pluralité de petits creux (17) qui sont petits en comparaison des évidements en forme de gorges et qui sont répartis dans les évidements en forme de gorges.

18. Implant dentaire selon la revendication 17, caractérisé en ce que la largeur des creux (17) a une valeur d'environ trois microns et leur profondeur est d'environ un micron.

19. Implant dentaire selon la revendication 1, caractérisé en ce que le corps d'implant comprend une zone collier (64, 74) qui est disposée entre les évidements en forme de gorges et une tête d'implant et qui couvre la périphérie du corps d'implant, en ce que la zone collier comprend à sa périphérie une pluralité de petits creux répartis spatialement (17), et en ce que le corps d'implant (77) comprend une zone de pied doucement polie (80) à son extrémité inférieure.

20. Implant dentaire selon les revendications 1 et 2, caractérisé en ce que la graduation des diamètres des sections est comprise entre 20 et 300 microns.

21. Méthode de fabrication d'un implant dentaire ayant un corps d'implant (30, 44) préformé présentant une symétrie de rotation et ayant une surface rugueuse sur laquelle une pluralité d'évidements en forme de gorges sont réalisés par une opération de retrait de matériau, la surface du corps d'implant étant divisée dans la direction de son axe longitudinal en un certain nombre de sections (45-50, 61),
caractérisé en ce que
les sections sont séparées les unes des autres par des bandes radiales étendues (52, 62), et en ce que sont formés par une opération de retrait de matériau dans chacune des sections (45-50, 61) à sa périphérie, une pluralité d'évidements en forme de gorges (20, 54, 63) s'étendant dans la direction de l'axe longitudinal du corps d'implant ou selon un angle aigu avec lui, et en ce que les évidements en forme de gorges ont un ordre de taille qui correspond à l'ordre de taille des ostéones dans le tissu du maxillaire qui sont attachés aux évidements en forme de gorges.

22. Méthode selon la revendication 21, caractérisée en ce que les sections sont préformées selon des diamètres qui sont dégressifs par pas successifs en allant vers le fond du corps d'implant pour former un cône (37, 40, 71).

23. Méthode selon la revendication 21, caractérisée en ce que, dans les bandes radiales étendues (52, 62), ladite opération de retrait pour produire les évidements en forme de gorges (20, 54, 63) est interrompue.

24. Méthode selon la revendication 22, caractérisée en ce que les bandes radiales étendues sont formées par les pas (37, 40, 71) entre les sections.

25. Méthode selon la revendication 22, caractérisée en ce que la graduation des diamètres est comprise entre 20 et 300 microns.

26. Méthode selon la revendication 21, caractérisée en ce que les évidements en forme de gorges (20) sont étroitement adjacents entre eux et ont un profil concave dont les bords se terminent à la périphérie des sections (37, 40, 45-50, 61, 71).

27. Méthode selon la revendication 21, caractérisée en ce que la largeur des évidements en forme de gorges (20) est comprise dans l'intervalle 20 à 300 microns, et en ce que la profondeur des évidements en forme de gorges (20) est comprise dans l'intervalle 10 à 150 microns.

28. Méthode selon la revendication 21, caractérisée en ce que une pluralité d'évidements en forme de creux (17) sont réalisés dans les évidements en forme de gorges (20) par une opération de retrait de matériau additionnelle, les évidements en forme de creux étant petits en comparaison des évidements en forme de gorges et étant répartis spatialement dans les évidements en forme de gorges.

29. Méthode selon les revendications 21 et 23, caractérisée en ce que un corps d'implant (36, 70) présentant une symétrie de rotation est préformé en degrés (37, 71, 85-90) pour les différents diamètres en utilisant un procédé de frittage, et en ce que dans chacune des étapes, une pluralité d'évidements en forme de gorges (38, 72, 95) sont réalisés par une opération de retrait de matériau, les évidements en forme de gorges s'étendant dans la direction de l'axe longitudinal du corps d'implant ou selon un angle aigu avec lui.

30. Méthode selon la revendication 29, caractérisée en ce que les évidements en forme de gorges (38, 72, 95) dans les degrés (37, 71, 85-90) ont essentiellement les mêmes dimensions sur toute la longueur du corps d'implant.

31. Méthode selon la revendication 29, caractérisée en ce que sur la longueur du corps d'implant dans au moins une partie des degrés (84-90) différentes structures de gorges et/ou évidements en forme de gorges de différentes tailles sont produits par une opération de retrait de matériau.

32. Méthode selon les revendications 22 et 24, caractérisée en ce que une pluralité de petits creux répartis spatialement sont implémentés dans les bandes (62, 73) par une opération de retrait de matériau.

33. Méthode selon l'une des revendications 21 à 31, caractérisée en ce que une zone collier (64, 74) est disposée au dessus des sections et couvre la périmètre du corps d'implant, et une pluralité de petits creux répartis spatialement (71) sont implémentés dans la zone collier par une opération de retrait de matériau..

34. Méthode selon l'une des revendications 21 à 33, caractérisée en ce que l'opération de retrait de matériau est effectuée par un rayon laser.

35. Méthode selon l'une des revendications 21 à 34, caractérisée en ce que l'opération de retrait de matériau est effectuée par une fraiseuse de précision.

36. Méthode selon l'une des revendications 21 à 34, caractérisée en ce que les évidements en forme de creux (17) sont produits par gravure.

37. Méthode selon la revendication 36, caractérisée en ce que la largeur des creux (17) a une valeur d'environ trois microns et leur profondeur est d'environ un micron.

38. Méthode selon la revendication 36, caractérisée en ce que l'opération de gravure est simultanément appliquée aux évidements en forme de gorges (20, 38, 63, 72, 95) et à la zone collier (64, 74) dont le périmètre couvre le corps d'implant.

39. Méthode selon la revendication 36, caractérisée en ce que l'opération de gravure est simultanément appliquée aux évidements en forme de gorges (20, 38, 63, 72, 95) et aux bandes radiales étendues (62, 73).

40. Méthode selon l'une des revendications 21 à 39, caractérisée en ce que l'opération de retrait de matériau est appliquée à un moule en coquille divisé qui est utilisé pour produire l'implant, en ce que le moule en coquille divisé a la forme d'un cylindre, d'un cône ou d'un cône à incréments avec une surface douce correspondant à la forme de l'implant à réaliser, et en ce qu'une représentation négative des structures de ladite surface est implémentée dans le moule à coquille divisé.
